# EUROPEAN PATENT SPECIFICATION

(11) **EP 4 028 861 B1**
(45) Date of publication and mention of the grant of the patent: **04.11.2026**
(21) Application number: 20765078.9
(22) Date of filing: 11.08.2020
(51) Int. Cl.: B62K 23/00, G06F 3/01, A61F 2/72, A61F 4/00, A61G 5/04, A61G 5/10, B62K 11/00

(54) **WEARABLE INTERFACE DEVICE FOR HANDS- FREE CONTROL OF POWER- DRIVEN VEHICLE**
TRAGBARE SCHNITTSTELLENVORRICHTUNG ZUR FREIHÄNDIGEN STEUERUNG EINES KRAFTFAHRZEUGS
DISPOSITIF D'INTERFACE PORTABLE POUR LA COMMANDE MAINS-LIBRES D'UN VÉHICULE MOTORISÉ

(30) Priority: 09.09.2019 LT 2019519
(43) Date of publication of application: 20.07.2022
(73) Proprietor: UAB "Chronus", 50281 Kaunas (LT)
(72) Inventor: GRISKA, Ricardas, 53456 Pypliai, Kauno r. sav. (LT)
(74) Representative: Pranevicius, Gediminas
(86) International application number: PCT/IB2020/057530
(87) International publication number: WO 2021/048656

(56) References cited:
- KR-A- 20040 084 055
- US-A1- 2012 283 929
- PAPI ENRICA ET AL: "Wearable technology for spine movement assessment: A systematic review", JOURNAL OF BIOMECHANICS, vol. 64, 7 October 2017 (2017-10-07), pages 186 - 197, XP085263594, ISSN: 0021-9290, DOI: 10.1016/J.JBIOMECH.2017.09.037
- BIOPAC SYSTEMS ET AL: "Goniometers & Torsiometers", 21 October 2014 (2014-10-21), XP055760808, Retrieved from the Internet <URL:https://www.biopac.com/wp-content/uploads/Goniometers-Torsiometers.pdf> [retrieved on 20201216]

## Description

### Field of the invention

The present invention relates to a wearable interface device for hands-free control of the vehicle or other power-driven equipment based on the movements of the user's spine.

### Background of the invention

In some situations, the user of the vehicle or other power-driven equipment does not have free use of his hands for operating the vehicle or other power-driven equipment. Such restrictions may occur due to the necessity of performing other functions with the use of hands as in industrial, military or aerospace operations or due to a limited upper limb mobility.

A wide range of systems and mechanisms are known for hands-free control of the vehicle by receiving user's inputs in order to control the vehicle. These include eye-tracking systems, voice-controlled systems, head-mounted servo control systems, sip/puff control systems, also control systems, that are based on sensing of head movements, shoulders movements, chin movements, sensing of dislocation of the centre of gravity, sensing of the user's body position, sensing of the user's body movements by measuring the rotation or tilting of the seat, sensing of the user's body movements by using pressure sensors positioned on the seat rest, on the back rest or on the feet rest of the vehicle. For example, the document US7275607 discloses a controller for providing user input of a desired direction of motion or orientation for a transporter. The controller has an input for receiving specification by a user of a value based on a detected body orientation of the user. User-specified input may be conveyed by the user using any of a large variety of input modalities, including: ultrasonic body position sensing; foot force sensing; handlebar lean; active handlebar; mechanical sensing of body position; and linear slide directional input. The document US7080710 discloses a wheelchair control sensor for controlling a powered wheelchair for spinal cord-injured persons, who are incapable of using their hands, using movement of shoulders. The document WO2019002597 refers to a control of an electric wheelchair, comprising an element for inputting commands and an adapter box for the transmission of data of the input element to an input/output module of the electric wheelchair, the data of the input element being transmissible to the adapter box wirelessly and the input element comprising a wearable computer system, where the wearable is a Smartglass. The document US7748490 discloses an electrically operable wheelchair including the hands-free input device, which includes the position detection assembly and a position detection device, provides input signals representative of desired wheelchair movements to the controller. The position detection device detects the position of the seat. Movement of the seat, rather than a joystick, creates the voltage signals which are then transmitted to the wheelchair controller. The document KR20040084055A describes an electrically operable wheelchair including the hands-free input device, control of which is based on the sensor means attached to the user's head, which makes it difficult to perform useful work or other functions like communication, observation of the surroundings. The document US2012283929A1 describes an electrically operable wheelchair including the hands-free input device, control of which is based on sensing of the user's body movements by using the pressure sensor sheets that are integrated in the backrest of the seat. Control management of the system requires a lot of attention from the user, because the user must constantly be in contact with the backrest of the seat, pressing the sensors with his shoulder blades, which greatly limits user's ability to perform other movements and the ability to freely use hands. The Journal of Biomechanics in its article "Wearable technology for spine movement assessment: A systematic review" (Journal of Biomechanics vol. 64 (2017) pages 186-197 discusses the use of wearable tools to track spine movement. Some sensors used in these tools have been described in Biopac Systems, Inc. product sheet "Goniometers & Torsiometers" of 21.10.2014.

Although these systems can be effective in specific applications, they can also have some limitations. The disadvantage of systems such as eye-tracking systems, voice-controlled systems, head-mounted servo control systems, sip/puff controlled systems, also control systems, that are based on sensing of head movements, chin movements, sensing of the user's body movements by measuring the rotation or tilting of the seat, sensing of the user's body movements by using pressure sensors positioned on the seat rest or on the back rest, is the requirement to focus on the system management, which makes it difficult to perform useful work or other functions like communication, observation of the surroundings, etc. The disadvantage of systems that are based on the sensing of the user's body movements by measuring the rotation or tilting of the seat or by using pressure sensors positioned on the feet rest is that performing such body movements generally requires the help of legs or hands while such operations can be difficult for users with limited lower limb mobility, or when hands are used for performing other functions. The disadvantage of systems that are based on the sensing of the user's body position or sensing of shoulders movements is the limitation of the movements needed to perform other tasks.

While above mentioned devices can be effective in limited applications, it would be eligible to provide wearable hands-free interface device, which minimally restricts the freedom of user motions and is based on intuitive and natural movements of the user's spine.

### Summary of invention

The present invention is defined by the independent claims. The dependent claims define further advantageous embodiments. It shall also be noted that the references made through this description to an "embodiment", "example", "aspect" or "configuration" may point to alternative aspects related to the invention but do not necessarily correspond to real realisations of it. The actual embodiments of the invention fall within the scope of the appended claims. It is to be understood that other examples can be practiced, and various modifications and structural changes can be made to these actual embodiments of the invention without departing from the scope of the claims, as would be understood by one of ordinary skill in the art.

The human spine is made of 33 individual vertebrae stacked one on top of the other. This spinal column provides the main support for the human body, muscles and bones. Flexible tendons and ligaments, and sensitive nerves contribute to spine movements.

A person who can control the spine can change the position of groups of vertebrae, wherein sensing of these changes can be used to control the vehicle or other power-driven equipment. The simplest natural movements of the spine are: leaning left and right in the frontal plane, leaning front-back in the sagittal plane and twist in the transverse plane.

An object of the present invention is a wearable interface device for hands-free control of the vehicle, which in some conditions overcomes the disadvantages of the prior art systems described above, and is configured to sense the user's spine movements in order to control the vehicle, receive input signals, based on said movements, and provide said signals directly to the vehicle controller or to a logic circuit of the signal processing unit, which is configured to process said input signals, generate control signals in response to said input signals and provide said control signals to the vehicle controller.

The wearable interface device, which is generally characterised by the user's spine movements in the frontal, sagittal and transverse planes, includes sensors that are arranged to measure angular spinal twist or lean, an equipment for positioning of said sensors on the user's body and a signal processing unit including a logic circuit, which is configured to receive inputs from sensors, generate control signals in response to said inputs and provide said control signals to the vehicle controller. The wearable interface device may be wired to the vehicle controller for a direct control or may include a wireless transceiver for a remote control.

If the vehicle controller is not intended for receiving wireless control signals, the vehicle can be additionally equipped with a communication module including a wireless transceiver and a logic circuit for communicating with the wearable interface device, decoding said control signals received from the wearable interface device and providing processed control signals to the vehicle controller.

Additionally a wearable interface device can be used with a variety of vehicles or other power-driven equipment.

### Brief description of the drawings

FIG. 1 is a simplified schematic view of one embodiment of the wearable interface device for hands-free control of the vehicle, provided with the rotary sensor;
FIG. 2 is a simplified detail schematic view of sensor means, provided with the rotary sensor and the signal processing unit of the wearable interface device for hands-free control of the vehicle;
FIG. 3 is a perspective view of a balancing personal vehicle according to the present invention wherein yaw is controlled by the wearable interface device provided with the rotary sensor;
FIG. 4A and 4B are simplified schematic views of the embodiments of the wearable interface device for hands-free control of the vehicle, provided with different sensor means;

### Detailed description of illustrative embodiments

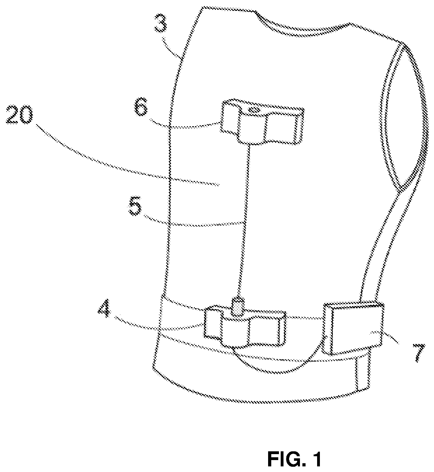

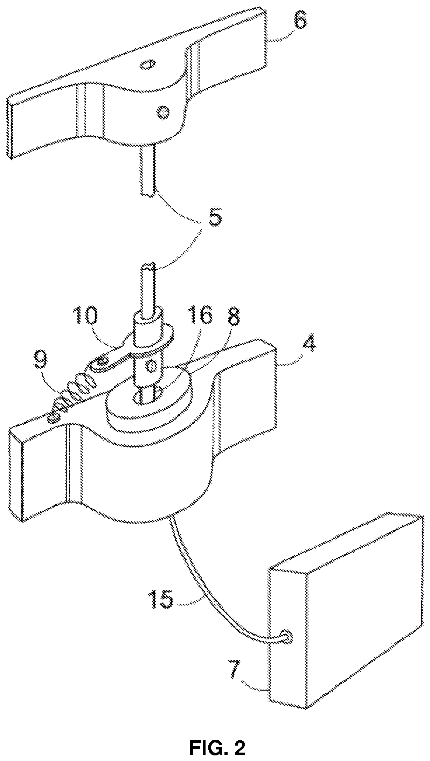

In the first embodiment, referring to Fig. 1 and 2, this invention relates to a wearable interface device 20, which is worn by the user 1, configured to provide yaw control signals, which are based on twist movements of the user's spine, to the vehicle controller, where the wearable interface device includes:
a rotary angle sensor 8, mounted in a housing 4;
an equipment for positioning said sensor on the user's body in a form of a vest 3, where the housing 4 of the sensor 8 is preferably fixed to the lower spinal part of the vest 3 at the vertebral level of Lumbar 1 to Lumbar 5 (L1-L5), in a manner where the shaft 16 of the sensor is relatively parallel to the user's spine;
a steering bracket 6, which is preferably fixed to the upper spinal part of the vest 3 at the vertebral level of Thoracic 1 to Thoracic 5 (T1-T5);
   a steering shaft 5, where one end of the steering shaft is fixed to the steering bracket 6 and the other end is fixed to the shaft 16 of the rotary angle sensor 8. The steering shaft is preferably flexible but twist-resistant;
a spring 9, which is provided to keep the shaft 16 of the sensor 8 in a respectively neutral position and to apply some counter force to the rotation of the steering shaft 5. The spring is connected to the housing 4 of said sensor at one end and to the lever 10, which is fixed on the steering shaft 5, at the other end. Torsional or conventional spring can be used.

To initiate a steering or yaw motion of the vehicle, the user twists his spine to a desired direction, which causes an angular change between upper and lower parts of the spine and in consequence results in a rotary motion of the rotary angle sensor 8. The rotary angle sensor 8 generates signals substantially proportional to the angular rotation of the shaft 5 and said signals, characterised as the user's inputs in order to control yaw of the vehicle, are provided to the vehicle controller. When the user releases the muscles and returns to the neutral (forward facing) position, the spring 9 helps to rotate the shaft 5 to its neutral position, and respectively the wearable interface device provides a control signal to the vehicle controller initiating a return to a neutral yaw position.

In the embodiment, where the input signals from the wearable interface device are not suitable for the vehicle controller, wearable interface device may include a signal processing unit 7, comprising:
a logic circuit for processing said user's inputs detected by the sensors, generating control signals in response to said inputs and providing said control signals to the vehicle controller;
a connector for data transfer.

In the embodiment, where the wearable interface device is not wired to the vehicle controller, the signal processing unit 7 further comprises:
a wireless transceiver for communicating with the vehicle controller;
a power module comprising a battery, a charging module and a connector for external power supply.

In the embodiment, where the vehicle controller is not intended for receiving wireless control signals from the signal processing unit 7 of the wearable interface device, the vehicle may be additionally equipped with a communication module 17, which then would be wired to the vehicle controller, the communication module 17 comprising:
a wireless transceiver for communicating with the wearable interface device;
a logic circuit for decoding said control signals received from the wearable interface device and providing processed control signals to the vehicle controller.

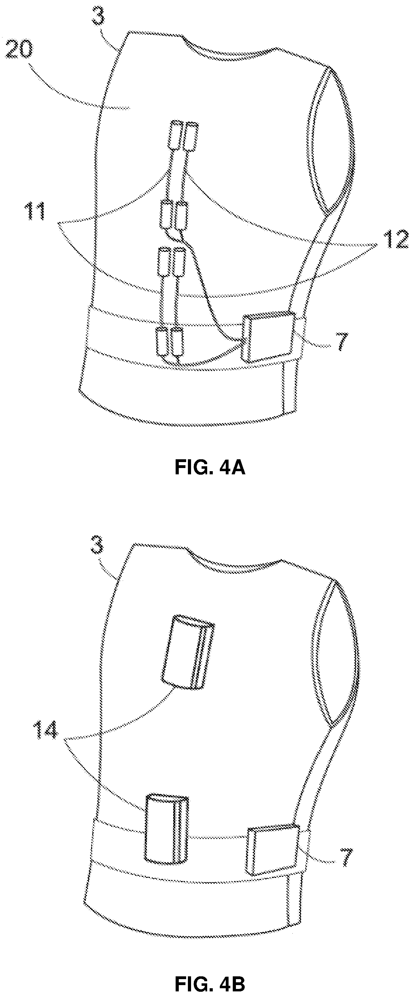

In the following embodiment, referring to Fig. 4A, the wearable interface device 20 includes at least one single axis electro-torsiometer 11 for sensing twist movements of the user's spine. For example, Biometrics Q series single axis electro-torsiometers that consist strain gauges could be used for sensing said twist movements. The single axis electro-torsiometer allows the measurement of rotation in one plane, for example, twist in the transverse plane. Axial rotation of one end block of the electro-torsiometer relative to the other end block along the longitudinal axis is measured from the single output channel. If the electro-torsiometer is bent in the sagittal plane or transverse plane, the output remains constant. In this embodiment one end block of the electro-torsiometer 11 is fixed to the lower spinal part of the vest 3 and another end block is fixed to the upper spinal part of the vest.

In some embodiments, the sensors are directly attached to the spinal area of the user's body by using a flexible adhesive, such as a suitable adhesive tape.

In the following embodiment, referring to Fig. 4A, the wearable interface device 20 additionally includes sensor means for sensing the movements of the user's spine in the sagittal plane or pitch, which is determined by leaning forward or backward, and sensing of the user's spine movements in the frontal plane or roll, which is determined by leaning to the right or to the left. For example, Biometrics SG series twin axis electro-goniometers 12 or other flex sensors could be used for sensing said movements. In this embodiment, yaw (in the case of the land vehicle - steering) of the vehicle can be controlled by signals based on twist movements of the user's spine, roll of the vehicle can be controlled by the signals based on lean movements of the user's spine in the frontal plane and pitch (in the case of the land vehicle - acceleration, speed and braking) of the vehicle can be controlled by the signals based on lean movements of the user's spine in the sagittal plane.

In some embodiments, the sensors of the wearable interface device are able to monitor the range and speed of at least the following movements of the spine: forward bending, backward bending, sideways bending to the right or left and twisting. The wearable interface device is able to sense combined movements.

In the following embodiment, referring to Fig. 4B, the wearable interface device 20 includes at least one 3D motion tracker 14, based on 3D accelerometers, which are capable of measuring pitch, roll and yaw angles of the user's spine or respectively angles in the sagittal, frontal and transverse planes. A 3D motion tracker can be described as a self-contained system that measures linear and angular motion with a triad of gyroscopes and a triad of accelerometers. The 3D motion trackers 14 are positioned on the user's body, preferably on the lower and upper parts of the user's spine or on the special equipment, such as the vest 3 as mentioned in the first embodiment, and are arranged to measure a range of motion, which is defined by the difference in the angles between the lower 3D motion tracker and the upper 3D motion tracker. For example, in this embodiment wireless 3D motion trackers from Xsens can be used.

In some embodiments, the signal processing unit 7 is designed to communicate wirelessly with a number of 3D motion trackers 14.

In the preferred embodiment, in the case of lost communication between the signal processing unit 7 and the vehicle controller, the vehicle controller is automatically set to a reference neutral position, and in the case of flight vehicles, the controller is automatically switched to an automatic (autopilot) mode.

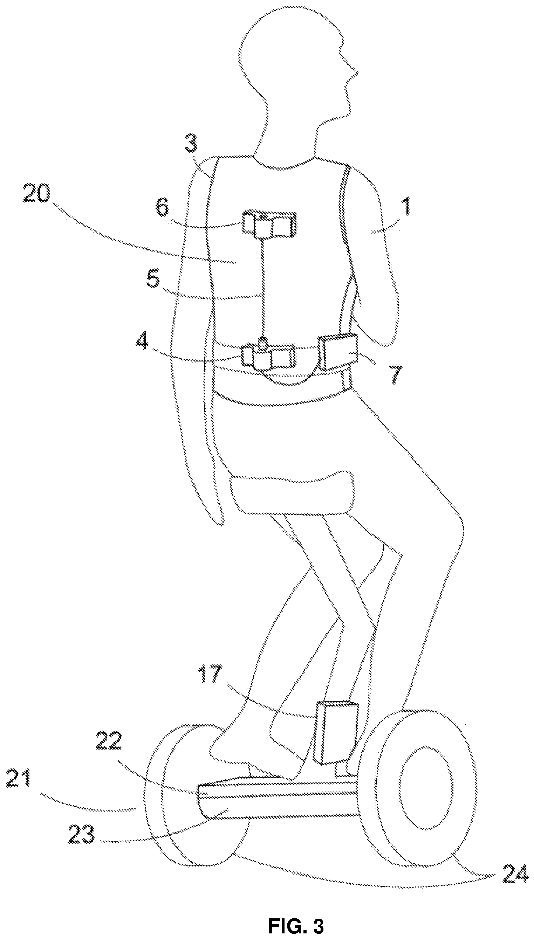

In the following embodiment, referring to Fig. 3, this invention relates to a balancing personal vehicle 21 comprising:
a platform 22 configured to support a load including the user 1;
a locomotion device 23 having two wheels 24, which are arranged substantially side-by-side at both sides of the platform 22, and electric motors (not shown) for driving the wheels, battery (not shown);
a vehicle controller (not shown) configured to control a locomotion device and to maintain a balanced state of the vehicle 21, where speed and acceleration is controlled by transferring a centre of gravity forward and backward and steering or yaw is controlled by the inputs from the wearable interface device 20 worn by the user 1;
the communication module 17 including a wireless transceiver for communicating with the wearable interface device and a logic circuit for decoding said control signals received from the wearable interface device and providing processed control signals to the vehicle controller.

In the following embodiment, various sensors can be provided for measuring angular change of the user's spine including, but not limited to, accelerometers, inclinometers, force or pressure sensors, soft sensors and rotary sensors. The sensors can be fixed directly on the user's body or on specially adapted clothes, harnesses or other similar equipment, such as the vest as mentioned in the preferred embodiment.

In some embodiments, the conventional user's detection sensor can be used to detect when the user gets on or off the vehicle. The user's detection sensor can be positioned in the seat or in the foot rest, or at other appropriate locations.

In some embodiments, the wearable interface device 20 can be used to control power-driven equipment like construction, military or space equipment.

In further embodiments, the wearable interface device 20 can be used to control exoskeletons or similar devices.

A number of embodiments of this invention were described above, although various other modifications and configurations may become apparent to those skilled in the art, thereby this invention is not limited by the above written embodiments, but within the scope of the appended claims.

## Claims

1. A wearable interface device for hands-free control of a vehicle, where the vehicle has a platform configured to support the load, including the user, a locomotion device coupled to the platform and arranged to propel the vehicle, and a vehicle controller configured to control the locomotion device according to the user input signals, wherein the wearable interface device (20) comprises:
sensor means for sensing at least one of twist movements of the user's spine, or lean movements of the user's spine in the frontal plane, sagittal and transverse planes;
means for supporting the sensor means on the user's spinal area comprising adhesive, harness or specially adapted clothes, such as a vest (3);
a signal processing unit (7) comprising a logic circuit, adapted to receive input signals from the sensor means, process said input signals, generate control signals in response to said input signals and provide said control signals to the vehicle's controller.

2. The device according to claim 1, wherein the sensor means comprise at least one sensor means such as a single axis electro-torsiometer (11) for sensing the twist movements of the user's spine, where control signals generated in response to input signals from said sensor means are used to control the yaw of the vehicle.

3. The device according to claim 1, wherein the sensor means comprise at least one sensor means such as an electro-goniometer (12) for sensing the lean movements of the user's spine in the frontal plane, where control signals generated in response to input signals from said sensor means are used to control the roll of the vehicle, and at least one sensor means for sensing the lean movements of the user's spine in the sagittal plane, where control signals generated in response to input signals from said sensor means are used to control the pitch or acceleration of the vehicle.

4. The device according to claim 1, wherein the sensor means comprise at least one 3D motion tracker (14) based on 3D accelerometers, which is capable of sensing the movements of the user's spine in the frontal, sagittal and transverse planes.

5. The device according to claim 2, wherein the sensor means configured to sense the twist movements of the user's spine for controlling the yaw of the vehicle comprise:
a rotary angle sensor (8) mounted in a housing (4);
a steering bracket (6);
a steering shaft (5), where one end of said shaft is fixed to the steering bracket (6) and the other end is fixed to a shaft (16) of the rotary angle sensor (8);
a spring (9), which is provided to keep the shaft (16) of the sensor (8) in a respectively neutral position and to apply some counter force to the rotation of the steering shaft (5), where the spring is connected to the housing (4) of the sensor at one end and to a lever (10), which is fixed on the steering shaft (5), at the other end.

6. The device according to claim 1, wherein the means for supporting the sensor means comprise an equipment in a form of the vest (3) for positioning the housing (4) of the rotary angle sensor (8) to the lower spinal part of the vest (3) at the vertebral level of Lumbar 1 to Lumbar 5 in a manner where the shaft (5) of the rotary angle sensor (8) is relatively parallel to the user's (1) spine, and the steering bracket (6) is fixed to the upper spinal part of the vest (3) at the vertebral level of Thoracic 1 to Thoracic 5.

7. The device according to claim 1, wherein the signal processing unit (7) further comprises:
a wireless transceiver for providing signals from the wearable interface device to the vehicle controller;
a power module, including a battery.

8. A system comprising the vehicle having a platform configured to support the load, including the user, a locomotion device coupled to the platform and arranged to propel the vehicle, a vehicle controller configured to control the locomotion device according to the user input signals, and a wearable interface device according to **any of** claims 1 to 7.

## Patentansprüche

1. Tragbare Schnittstellenvorrichtung zur freihändigen Steuerung eines Fahrzeugs, wobei das Fahrzeug eine Plattform, die dafür ausgelegt ist, die Last, einschließlich des Benutzers, zu tragen, eine Fortbewegungsvorrichtung, die mit der Plattform gekoppelt und dafür ausgelegt, das Fahrzeug anzutreiben, und eine Fahrzeugsteuerung, die dafür ausgelegt ist, die Fortbewegungsvorrichtung gemäß den Benutzereingabesignalen zu steuern, aufweist, wobei die tragbare Schnittstellenvorrichtung (20) Folgendes umfasst:
Sensormittel zum Erfassen mindestens eines von Drehbewegungen der Wirbelsäule des Benutzers oder Beugebewegungen der Wirbelsäule des Benutzers in der Frontalebene, der Sagittal- und der Transversalebene;
Mittel zum Halten der Sensormittel im Wirbelsäulenbereich des Benutzers, die Haftmittel, einen Tragegurt oder speziell angepasste Kleidung, wie eine Weste (3), umfassen;
eine Signalverarbeitungseinheit (7), die eine Logikschaltung umfasst, die dafür ausgelegt ist, Eingabesignale von den Sensormitteln zu empfangen, die Eingabesignale zu verarbeiten, Steuersignale als Reaktion auf die Eingabesignale zu erzeugen und die Steuersignale der Fahrzeugsteuerung bereitzustellen.

2. Vorrichtung nach Anspruch 1, wobei die Sensormittel mindestens ein Sensormittel, wie beispielsweise ein einachsiges Elektrotorsiometer (11), zum Erfassen der Drehbewegungen der Wirbelsäule des Benutzers umfassen, wobei die Steuersignale, die als Reaktion auf die Eingabesignale von den Sensormitteln erzeugt werden, dafür verwendet werden, das Gieren des Fahrzeugs zu steuern.

3. Vorrichtung nach Anspruch 1, wobei die Sensormittel mindestens ein Sensormittel, wie beispielsweise ein Elektrogoniometer (12), zum Erfassen der Beugebewegungen der Wirbelsäule des Benutzers in der Frontalebene umfassen, wobei Steuersignale, die als Reaktion auf Eingabesignale von den Sensormitteln erzeugt werden, dafür verwendet werden, das Wanken des Fahrzeugs zu steuern, und mindestens ein Sensormittel zum Erfassen der Beugebewegung der Wirbelsäule des Benutzers in der Sagittalebene umfassen, wobei Steuersignale, die als Reaktion auf Eingabesignale von den Sensormitteln erzeugt werden, dafür verwendet werden, das Nicken oder die Beschleunigung des Fahrzeugs zu steuern.

4. Vorrichtung nach Anspruch 1, wobei die Sensormittel mindestens eine auf 3D-Beschleunigungsmessern basierende 3D-Bewegungsverfolgungseinrichtung (14) umfassen, die in der Lage ist, die Bewegungen der Wirbelsäule des Benutzers in der Frontal-, der Sagittal- und der Transversalebene zu erfassen.

5. Vorrichtung nach Anspruch 2, wobei die Sensormittel, die dafür ausgelegt sind, die Drehbewegungen der Wirbelsäule des Benutzers zum Steuern des Gierens des Fahrzeugs zu erfassen, Folgendes umfassen:
einen Drehwinkelsensor (8), der in einem Gehäuse (4) angebracht ist;
einen Lenkungsbock (6);
eine Lenkwelle (5), wobei ein Ende der Welle an dem Lenkungsbock (6) befestigt ist und das andere Ende an einer Welle (16) des Drehwinkelsensors (8) befestigt ist;
eine Feder (9), die dafür vorgesehen ist, die Welle (16) des Sensors (8) in einer jeweils neutralen Stellung zu halten und eine gewisse Gegenkraft gegen die Drehung der Lenkwelle (5) auszuüben, wobei die Feder an einem Ende mit dem Gehäuse (4) des Sensors und an dem anderen Ende mit einem Hebel (10), der an der Lenkwelle (5) befestigt ist, verbunden ist.

6. Vorrichtung nach Anspruch 1, wobei die Mittel zum Halten der Sensormittel eine Ausrüstung in Form der Weste (3) zur Positionierung des Gehäuses (4) des Drehwinkelsensors (8) am unteren Wirbelsäulenteil der Weste (3) auf der Wirbelhöhe von Lendenwirbel 1 bis Lendenwirbel 5 in einer Weise, dass die Welle (5) des Drehwinkelsensors (8) vergleichsweise parallel zu der Wirbelsäule des Benutzers (1) ist, umfassen und der Lenkungsbock (6) am oberen Wirbelsäulenteil der Weste (3) auf der Wirbelhöhe von Brustwirbel 1 bis Brustwirbel 5 befestigt ist.

7. Vorrichtung nach Anspruch 1, wobei die Signalverarbeitungseinheit (7) ferner Folgendes umfasst:
einen Drahtlos-Sendeempfänger zum Bereitstellen von Signalen von der tragbaren Schnittstellenvorrichtung an die Fahrzeugsteuerung;
ein Leistungsmodul, das eine Batterie umfasst.

8. System, das das Fahrzeug, das eine Plattform aufweist, die dafür ausgelegt ist, die Last, einschließlich des Benutzers, zu tragen, eine Fortbewegungsvorrichtung, die mit der Plattform gekoppelt und dafür ausgelegt, das Fahrzeug anzutreiben, eine Fahrzeugsteuerung, die dafür ausgelegt ist, die Fortbewegungsvorrichtung gemäß den Benutzereingabesignalen zu steuern, und eine tragbare Schnittstellenvorrichtung nach einem der Ansprüche 1 bis 7 umfasst.

## Revendications

1. Dispositif d'interface portable pour le contrôle mains libres d'un véhicule, dans lequel le véhicule comporte une plateforme configurée pour supporter la charge, y compris l'utilisateur, un dispositif de locomotion couplé à la plateforme et agencé pour propulser le véhicule, et un contrôleur de véhicule configuré pour contrôler le dispositif de locomotion en fonction des signaux d'entrée de l'utilisateur, dans lequel le dispositif d'interface portable (20) comprend :
des moyens à capteur permettant de détecter au moins un mouvement parmi des mouvements de torsion de la colonne vertébrale de l'utilisateur et des mouvements d'inclinaison de la colonne vertébrale de l'utilisateur dans un plan frontal, un plan sagittal et un plan transversal ;
des moyens pour supporter les moyens à capteur sur la zone spinale de l'utilisateur, comprenant un adhésif, un harnais ou des vêtements spécialement adaptés, tels qu'un gilet (3) ;
une unité de traitement de signal (7) comprenant un circuit logique, adaptée pour recevoir des signaux d'entrée provenant des moyens à capteur, pour traiter lesdits signaux d'entrée, pour générer des signaux de contrôle en réponse auxdits signaux d'entrée, et pour fournir lesdits signaux de contrôle au contrôleur du véhicule.

2. Dispositif selon la revendication 1, dans lequel les moyens à capteur comprennent au moins un moyen à capteur tel qu'un électro-torsiomètre mono-axial (11) permettant de détecter les mouvements de torsion de la colonne vertébrale de l'utilisateur, dans lequel les signaux de contrôle générés en réponse aux signaux d'entrée provenant dudit moyen à capteur sont utilisés pour contrôler les mouvements de lacet du véhicule.

3. Dispositif selon la revendication 1, dans lequel les moyens à capteur comprennent au moins un moyen à capteur tel qu'un électro-goniomètre (12) permettant de détecter les mouvements d'inclinaison de la colonne vertébrale de l'utilisateur dans le plan frontal, dans lequel les signaux de contrôle générés en réponse aux signaux d'entrée provenant dudit moyen à capteur sont utilisés pour contrôler les mouvements de roulis du véhicule, et au moins un moyen à capteur permettant de détecter les mouvements d'inclinaison de la colonne vertébrale de l'utilisateur dans le plan sagittal, dans lequel les signaux de contrôle générés en réponse aux signaux d'entrée provenant dudit moyen à capteur sont utilisés pour contrôler les mouvements de tangage ou l'accélération du véhicule.

4. Dispositif selon la revendication 1, dans lequel les moyens à capteur comprennent au moins un traceur de mouvement 3D (14) sur la base d'accéléromètres 3D, qui est capable de détecter les mouvements de la colonne vertébrale de l'utilisateur dans le plan frontal, le plan sagittal et le plan transversal.

5. Dispositif selon la revendication 2, dans lequel les moyens à capteur configurés pour détecter les mouvements de torsion de la colonne vertébrale de l'utilisateur afin de contrôler les mouvements de lacet du véhicule comprennent :
un capteur d'angle de rotation (8) monté dans un boîtier (4) ;
un support de direction (6) ;
un axe de direction (5), dans lequel une extrémité dudit axe est fixée au support de direction (6) et l'autre extrémité est fixée à un axe (16) du capteur d'angle de rotation (8) ;
un ressort (9), qui est pourvu pour maintenir l'axe (16) du capteur (8) dans une position neutre respective et pour appliquer une certaine contre-force à la rotation de l'axe de direction (5), dans lequel le ressort est connecté au boîtier (4) du capteur à une extrémité et à un levier (10) fixé sur l'axe de direction (5) à l'autre extrémité.

6. Dispositif selon la revendication 1, dans lequel les moyens de support des moyens à capteur comprennent un équipement sous la forme d'un gilet (3) pour positionner le boîtier (4) du capteur d'angle de rotation (8) sur la partie spinale inférieure du gilet (3) au niveau des vertèbres lombaires L1 à L5, de manière à ce que l'axe (5) du capteur d'angle de rotation (8) soit relativement parallèle à la colonne vertébrale de l'utilisateur (1), et le support de direction (6) est fixé à la partie spinale supérieure du gilet (3) au niveau des vertèbres thoraciques T1 à T5.

7. Dispositif selon la revendication 1, dans lequel l'unité de traitement de signal (7) comprend en outre :
un émetteur-récepteur sans fil destiné à fournir des signaux provenant du dispositif d'interface portable au contrôleur de véhicule ;
un module d'alimentation incluant une batterie.

8. Système comprenant le véhicule comportant une plateforme configurée pour supporter la charge, y compris l'utilisateur, un dispositif de locomotion couplé à la plateforme et agencé pour propulser le véhicule, un contrôleur de véhicule configuré pour contrôler le dispositif de locomotion en fonction des signaux d'entrée de l'utilisateur, et un dispositif d'interface portable selon l'une quelconque des revendications 1 à 7.
